(19) Europäisches Patentamt
European Patent Office
Office européen des brevets

(11) **EP 1 054 688 B1**

(12) **FASCICULE DE BREVET EUROPEEN**

(45) Date de publication et mention
de la délivrance du brevet:
**30.06.2004 Bulletin 2004/27**

(21) Numéro de dépôt: **99904919.0**

(22) Date de dépôt: **19.02.1999**

(51) Int Cl.[7]: **A61K 39/00**, C07K 16/28,
C07K 14/705, C12Q 1/68

(86) Numéro de dépôt international:
**PCT/FR1999/000386**

(87) Numéro de publication internationale:
**WO 1999/042128 (26.08.1999 Gazette 1999/34)**

(54) **METHODE DE SELECTION DE TUMEURS EXPRIMANT HLA-G, SENSIBLES A UN TRAITEMENT ANTICANCEREUX ET SES APPLICATIONS**

AUSWAHLVERFAHREN VON HLA-G EXPRIMIERENDEN TUMOREN, DIE DURCH ANTIKREBSMITTEL BEHANDELBAR SIND, UND DEREN VERWENDUNGEN

METHOD FOR SELECTING TUMOURS EXPRESSING HLA-G, SENSITIVE TO ANTICANCER TREATMENT AND USES

(84) Etats contractants désignés:
**BE CH DE DK ES FR GB IE IT LI NL SE**

(30) Priorité: **20.02.1998 FR 9802071**
**24.07.1998 FR 9809470**

(43) Date de publication de la demande:
**29.11.2000 Bulletin 2000/48**

(73) Titulaire: **COMMISSARIAT A L'ENERGIE ATOMIQUE**
**75015 Paris Cédex 15 (FR)**

(72) Inventeurs:
• **CAROSELLA, Edgardo, Delfino**
**F-75016 Paris (FR)**
• **DAUSSET, Jean**
**F-75007 Paris (FR)**
• **MOREAU, Philippe**
**F-91170 Viry-Chatillon (FR)**
• **PAUL, Pascale**
**F-75010 Paris (FR)**
• **ROUAS-FREISS, Nathalie**
**F-75013 Paris (FR)**

(74) Mandataire: **Vialle-Presles, Marie José et al**
**Cabinet ORES,**
**36,rue de St Pétersbourg**
**75008 Paris (FR)**

(56) Documents cités:
EP-A- 0 563 627            EP-A- 0 677 582
WO-A-96/31604

• AMIOT ET AL: "HLA - G transcription studies during the different stages of normal and malignant hematopoiesis" TISSUE ANTIGENS, vol. 48, no. 5, 1 novembre 1996, pages 609-614, XP002086255
• AMIOT ET AL: "Distribution of HLA - G alternative mRNAs including soluble forms in normal lymphocytes and in lymphoid cell-derived leukemia" EUROPEAN JOURNAL OF IMMUNOGENETICS, vol. 23, no. 4, 1 août 1996, pages 311-320, XP002086256
• P.PAUL ET AL.: "HLA-G EXPRESSION IN MELANOMA: A WAY FOR TUMOR CELLS TO ESCAPE FROM IMMUNOSURVEILLANCE" PROCEEDINGS OF NATURAL ACADEMY OF SCIENCES, USA, vol. 95, avril 1998, pages 4510-4515, XP002100587
• Y. YANG ET AL.: "EXPRESSION OF HLA-G IN HUMAN MONONUCLEAR PHAGOCYTES AND SELECTIVE INDUCTION BY IFN-GAMMA" THE JOURNAL OF IMMUNOLOGY, vol. 156, 1996, pages 4224-4231, XP002100588
• MOREAU ET AL: "Transcrit diff rentiels du g ne du CMH" COMPTES RENDUS DES SEANCES DE L'ACADEMIE DES SCIENCES SERIE III: SCIENCES DE LA VIE, vol. 318, 1 janvier 1995, pages 837-842, XP002086257 cité dans la demande
• KIRSZENBAUM ET AL: "An alternatively spliced form of HLA-G mRNA" PROCEEDINGS OF THE NATIONAL ACADEMY OF SCIENCES OF USA, vol. 91, 1 mai 1994, pages 4209-4213, XP002086258 cité dans la demande

- **PEREZ ET AL: "The CD94/NKG2-A inhibitory receptor complex is involved in natural killer cell-mediated recognition of cells expressing HLA-G1" JOURNAL OF IMMUNOLOGY, vol. 158, no. 12, 15 juin 1997, pages 5736-5743, XP002086259**
- **ROUAS ET AL: "The alpha1 domain of HLA-G1 and HLA-G2 inhibits cytotoxicity induced by natural killer cells: is HLA - G the public ligand for natural killer cell inhibitory receptors?" PROCEEDINGS OF THE NATIONAL ACADEMY OF SCIENCES OF USA, vol. 94, 1 mai 1997, pages 5249-5254, XP002086260 cité dans la demande**
- **ROUAS ET AL: "Direct evidence to support the role of HLA - G in protecting the fetus from maternal uterine natural killer cytolysis" PROCEEDINGS OF THE NATIONAL ACADEMY OF SCIENCES OF USA, vol. 94, 1 octobre 1997, pages 11520-11525, XP002086261**

Remarques:

Le dossier contient des informations techniques présentées postérieurement au dépôt de la demande et ne figurant pas dans le présent fascicule.

## Description

La présente invention est relative à une méthode de sélection de tumeurs solides sensibles à un traitement anticancéreux, qui inhibe ou prévient l'activité HLA-G desdites tumeurs solides et ses applications.

Les antigènes du complexe majeur d'histocompatibilité (CMH), se divisent en plusieurs classes, les antigènes de classe I (HLA-A, HLA-B et HLA-C) qui présentent 3 domaines globulaires ($\alpha$1, $\alpha$2 et $\alpha$3), et dont le domaine $\alpha$3 est associé à la $\beta$2 microglobuline, les antigènes de classe II (HLA-DP, HLA-DQ et HLA-DR) et les antigènes de classe III (complément).

Les antigènes de classe I comprennent, outre les antigènes précités, d'autres antigènes, dits antigènes de classe I non classiques, et notamment les antigènes HLA-E, HLA-F et HLA-G ; ce dernier, en particulier, est exprimé par les trophoblastes extravilleux du placenta humain normal et les cellules épithéliales thymiques.

La séquence du gène HLA-G (gène HLA-6.0) a été décrite par GERAGHTY et al., (Proc. Natl. Acad. Sci. USA, 1987, 84, 9145-9149) : il comprend 4396 paires de bases et présente une organisation intron/exon homologue à celle des gènes HLA-A, -B et -C. De manière plus précise, ce gène comprend 8 exons, 7 introns et une extrémité non traduite 3' ; les 8 exons correspondent respectivement à : exon 1 : séquence signal, exon 2 : domaine extracellulaire $\alpha$1, exon 3 : domaine extracellulaire $\alpha$2, exon 4 : domaine extracellulaire $\alpha$3, exon 5 : région transmembranaire, exon 6 : domaine cytoplasmique I, exon 7 : domaine cytoplasmique II (non traduit), exon 8 : domaine cytoplasmique III (non traduit) et région 3' non traduite (GERAGHTY et al., précité; ELLIS et al., J. Immunol., 1990, 144, 731-735 ; KIRSZENBAUM M. et al., *Oncogeny of hematopoiesis. Aplastic anemia* Eds. E. Gluckman, L. Coulombel, Colloque INSERM/John Libbey Eurotext Ltd). Toutefois le gène HLA-G diffère des autres gènes de classe I, en ce que le codon de terminaison de traduction, en phase, est localisé au niveau du deuxième codon de l'exon 6 ; en conséquence, la région cytoplasmique de la protéine codée par ce gène HLA-6.0 est considérablement plus courte que celle des régions cytoplasmiques des protéines HLA-A, -B et -C.

Ces antigènes HLA-G sont essentiellement exprimés par les cellules cytotrophoblastiques du placenta et sont considérés comme jouant un rôle dans la protection du foetus (absence de rejet par la mère). En outre, dans la mesure où l'antigène HLA-G est monomorphique, il peut également être impliqué dans la croissance ou la fonction des cellules placentaires (KOVATS et al., Science, 1990, 248, 220-223).

D'autres recherches concernant cet antigène non classique de classe I (ISHITANI et al., Proc. Natl. Acad. Sci. USA, 1992, 89, 3947-3951) ont montré que le transcrit primaire du gène HLA-G peut être épissé de plusieurs manières et produit au moins 3 ARNm matures distincts : le transcrit primaire d'HLA-G fournit une copie complète (G1) de 1 200 pb, un fragment de 900 pb (G2) et un fragment de 600 pb (G3).

Le transcrit G1 ne comprend pas l'exon 7 et correspond à la séquence décrite par ELLIS et al. (précité), c'est-à-dire qu'il code une protéine qui comprend une séquence leader, trois domaines externes, une région transmembranaire et une séquence cytoplasmique. L'ARNm G2 ne comprend pas l'exon 3, c'est-à-dire qu'il code une protéine dans laquelle les domaines $\alpha$1 et $\alpha$3 sont directement joints ; l'ARNm G3 ne contient ni l'exon 3, ni l'exon 4, c'est-à-dire qu'il code une protéine dans laquelle le domaine $\alpha$1 et la séquence transmembranaire sont directement joints.

L'épissage qui prévaut pour l'obtention de l'antigène HLA-G2 entraîne la jonction d'une adénine (A) (provenant du domaine codant $\alpha$1) avec une séquence AC (issue du domaine codant $\alpha$3), ce qui entraîne la création d'un codon AAC (asparagine) à la place du codon GAC (acide aspartique), rencontré au début de la séquence codant le domaine $\alpha$3 dans HLA-G1.

L'épissage généré pour l'obtention de HLA-G3 n'entraîne pas la formation d'un nouveau codon dans la zone d'épissage.

Les Auteurs de cet article ont également analysé les différentes protéines exprimées : les 3 ARNm sont traduits en protéine dans la lignée cellulaire .221-G.

Moreau et al. (C.R. Acad. Sci., Sciences de la vie/Lifesciences, 1995; 318:837-842) décrit (cf. pages 838 et 839, matériel et méthodes) une méthode d'établissement du profil de transcription HLA-G basée sur l'extraction de l'ARN de cellules du foetus humain, suivie de la transcription inverse et de l'amplification par des amorces spécifiques de HLA-G (tableau I). Les fragments obtenus sont analysés par électrophorèse et permettent d'identifier le profil de transcription HLA-G des cellules foetales.

Yang et al. (The Journal of Immunology, 1996, 156: 4224-4231) décrit (cf. pages 4225, matériel et méthodes) une méthode immunohistochimique permettant la détection de l'expression de l'antigène HLA-G sur des cellules cytrophoblastiques par un anticorps monoclonal.

Rouas et al. (PNAS, vol. 94, pp. 5249-5254, 1997) décrit (cf. pages 5250, deuxième colonne, deuxième alinéa) une méthode de détection des antigènes HLA-G1 et HLA-G2 par immunoprécipitation effectuée sur des cellules transformées par transfection et exprimant HLA-G1 ou HLA-G2.

Certains des Inventeurs ont montré l'existence d'autres formes epissées d'ARNm d'HLA-G : le transcrit HLA-G4, qui n'inclut pas l'exon 4 ; le transcrit HLA-G5, qui inclut l'intron 4, entre les exons 4 et 5, provoquant ainsi une modification du cadre de lecture, lors de la traduction de ce transcrit et en particulier l'apparition d'un codon stop, après l'acide aminé 21 de l'intron 4 ; et le transcrit HLA-G6, possédant l'intron 4, mais ayant perdu l'exon 3 (KIRSZENBAUM M. et al., *Proc. Natl. Acad. Sci.* USA, 1994, 91, 4209-4213 ; Demande Européenne EP 0 677 582 ; KIRSZENBAUM M. et al., *Human Immunol.,* 1995, 43, 237-241 ; MOREAU P. et al., *Human Immunol.* 1995, 43, 231-236) ; ils ont également montré que ces différents transcrits sont exprimés dans plusieurs types de cellules humaines foetales et adultes, notamment dans les lymphocytes (KIRSZENBAUM M. et al., *Human Immunol*., 1995, précité ; MOREAU P. et al., *Human Immunol.* 1995, précité).

Certains des Inventeurs ont également montré que les cellules NK n'expriment aucun transcrit HLA-G (TEYSSIER M. et al., *Nat. Immunol.,* 1995, 14, 262-270 ; MOREAU P. et al., *Human Immunol*., 1997, 52, 41-46).

Il existe donc au moins 6 ARNms HLA-G différents qui codent potentiellement 6 isoformes protéiques d'HLA-G, dont 4 membranaires (HLA-G1, G2, G3 et G4) et 2 solubles (G5, G6).

Bien que le foetus puisse être considéré comme une semiallogreffe, les cellules foetales survivent et ne sont pas rejetées par la mère ; il est apparu que les molécules HLA-G, exprimées à la surface des trophoblastes protègent les cellules foetales de la lyse par les cellules *natural killer* (NK) maternelles de la decidua utérine et du sang périphérique (CAROSELLA E.D. et al., C.R. Acad. Sci., 318, 827-830 ; CAROSELLA E.D. et al ; *Immunol. Today,* 1996, 407-409 ; ROUAS-FREISS N. et al., *PNAS*, 1997, 94, 5249-5254).

Des études antérieures ont montré que l'expression des molécules HLA-G à la surface de cellules cibles transfectées permet de protéger lesdites cellules cibles de l'activité lytique des cellules NK de la couche déciduale de l'endomètre maternel (CHUMBLEY G. et al., *Cell Immunol*., 1994, 155, 312-322 ; DENIZ G. et al., *J. Immunol*., 1994, 152, 4255-4261 ; ROUAS-FREISS N. et al., Proc. Natl. Acad. Sci., 1997, 94; 5249-5254). Il est à noter que ces cellules cibles sont obtenues par transfection avec des vecteurs comprenant soit l'ADN génomique de HLA-G, générant potentiellement tous les transcrits alternatifs, soit avec des vecteurs contenant les ADNc HLA-G1 et HLA-G2 codant les isoformes protéiques HLA-G1 et HLA-G2 (Demande de Brevet Européen n° 0 677 582 et Demande PCT/FR98/00333).

Les cellules NK expriment des récepteurs des molécules du CMH de classe I (*killer inhibitory receptors* ou KIR ou NKIR pour récepteurs inhibiteurs NK), qui sont responsables de l'inhibition de la cytotoxicité, lorsque ces molécules HLA, agissant comme ligands, sont reconnues par ces récepteurs ; par exemple, N. ROUAS-FREISS et al. (Proc. Natl. Acad. Sci., 1997, **94**, 5249-5254) ont montré que l'expression de HLA-G protégeait de la lyse, les cellules cibles K562 (lignée cellulaire érythroleucémique humaine), transfectées par les isoformes HLA-G1 et G2. Ces cellules sont habituellement sensibles aux cellules NK.

Ces résultats attestent du rôle fondamental de la molécule HLA-G en tant qu'antigène d'immunotolérance. Ces résultats ont été élargis à l'ensemble des isoformes membranaires. Les ADNc codant pour les isoformes HLA-G1, G2, G3, G4 exprimés après transfection dans différents types cellulaires, en particulier, les cellules K562 et les cellules tumorales M8 transfectées inhibent les fonctions cytotoxiques NK et CTL.

Compte tenu du rôle important que peut jouer la molécule HLA-G, les Inventeurs, poursuivant leurs travaux ont plus particulièrement étudiés les cellules tumorales et se sont notamment donnés pour but de pourvoir à des outils de sélection des tumeurs solides sensibles à un traitement qui inhibe les antigènes HLA-G, présents notamment sur certaines tumeurs.

La présente invention a pour objet une méthode d'établissement du profil de transcription HLA-G d'une tumeur solide en vue de la sélection d'un traitement adapté à ladite tumeur et/ou en vue de la surveillance de l'évolution de ladite tumeur, caractérisée en ce qu'elle comprend :

(i) l'extraction de l'ARNm, à partir d'un échantillon tumoral; on peut utiliser notamment, une méthode modifiée de Chomczynski et Sacchi, en utilisant le réactif RNA NOW (Ozyme, France) ;
(ii) la transcription inverse (RT) dudit ARN ;
(iii) les amplifications successives ou concomitantes des ADNc obtenus en (ii), en présence d'amorces spécifiques de chaque isoforme d'HLA-G et l'analyse des produits d'amplification obtenus, par électrophorèse et/ou hybridation spécifique et
(v) l'établissement du profil de transcription HLA-G dudit échantillon.

De manière préférée, les transcriptions inverses sont amorcées avec des oligo-dT sur de l'ARNm, préalablement dénaturé, par exemple à 65°C, en présence d'une transcriptase inverse, telle que la transcriptase inverse M-MLV (Gibco-BRL, Life technologies).

Également de manière préférée, l'amplification des ADNc est réalisée par polymérisation en chaîne (PCR), en utilisant des amorces spécifiques des différentes isoformes d'HLA-G, conformément aux Tableaux suivants :

| Amorces | Séquences nucléotidiques | Températures d'hybridation (°C) | Isoformes amplifiées |
|---|---|---|---|
| G.257 | 5'-GGAAGAGGAGACACGGAACA | 61 | G1, G2, G3, G4, G5, G6 |
| G3.U | 5'-GGCTGGTCTCTGCACAAAGAGA | | |
| G.526 | 5'-CCAATGTGGCTGAACAAAGG | 61 | G1, G4, G5 |
| G3.U | 5'-GGCTGGTCTCTGCACAAAGAGA | | |
| G.-3-4 | 5'-ACCAGAGCGAGGCCAAGCAG | 65 | G3 |
| G3.U | 5'-GGCTGGTCTCTGCACAAAGAGA | | |
| G.-3 | 5'-ACCAGAGCGAGGCCAACCCC | 65 | G2, G6 |
| G3.U | 5'-GGCTGGTCTCTGCACAAAGAGA | | |
| G.-3 | 5'-ACCAGAGCGAGGCCAACCCC | 61 | G6 |
| G.i4b | 5'-AAAGGAGGTGAAGGTGAGGG | | |
| G.526 | 5'-CCAATGTGGCTGAACAAAGG | 61 | G5 |
| G.i4b | 5'-AAAGGAGGTGAAGGTGAGGG | | |

| Sondes | Séquences nucléotidiques | Températures d'hybridation (°C) | Isoformes détectées |
|---|---|---|---|
| GR | 5'-GGTCTGCAGGTTCATTCTGTC | 60 | HLA-G1, G2, G3, G4, G5, G6 |
| G.647 F | 5'-CCACCACCCTGTCTTTGACT | 60 | HLA-G1, G2,G5, G6 |
| G.I4 F | GAGGCATCATGTCTGTTAGG | 55 | HLA-G5, G6 |
| G.927 F | 5'-ATCATGGGTATCGTTGCTGG | 55 | HLA-G1, G2, G3, G4, G5 et G6 |

Les Inventeurs ont trouvé, de manière surprenante, qu'au moins certaines tumeurs solides expriment l'antigène HLA-G et ont montré que cet antigène HLA-G joue un rôle fonctionnel dans la protection des cellules tumorales (tumeurs solides) de la destruction par les cellules NK. Ils ont en outre montré la présence effective de certaines des isoformes d'HLA-G à la surface desdites cellules tumorales.

Toutefois, également de manière surprenante, selon les lignées tumorales, le profil HLA-G (transcrits et protéines) sera différent.

Par exemple, dans certaines lignées de mélanome, on peut observer la présence des isoformes HLA-G2/G4 et G3, qui protègent ces lignées de la lyse cellulaire induite par les cellules NK, comme le fait l'isoforme HLA-G 1, dans d'autres lignées.

Dans d'autres lignées, l'ensemble des transcrits HLA-G sont détectés. La forme protéique HLA-G 1 est détectée par immunofluorescence avec un anticorps anti-HLA-G et inhibe la lyse NK.

L'analyse de biopsies de patients atteints de mélanomes révèle un taux de transcrits élevé HLA-G dans certaines tumeurs (primitives et métastases) associé à une forte expression de la protéine HLA-G1 détectable en immunohistochimie sur coupes congelées avec un anticorps anti-HLA-G1.

Cette transcription et expression élevée HLA-G est spécifique du tissu tumoral et n'est pas détectée dans le tissu sain.

Dans certains mélanomes, on observe une dissociation de la transcription des isoformes solubles (G5) et membranaires. L'analyse des patients révèle 4 profils de transcription et d'expression HLA-G.

| Profils de transcription | Formes membranaires HLA-G1, G2, G3, G4 | Formes solubles |
|---|---|---|
| Profil 1 | - | - |
| 2 | ++ | - |
| 3 | - | ++ |
| 4 | ++ | ++ |

L'expression de la protéine soluble est détectée en immunohistochimie sur les patients présentant le profil P4.

La présente invention a également pour objet une méthode d'établissement du profil d'expression HLA-G d'une tumeur solide, en vue de la sélection d'un traitement adapté à ladite tumeur et/ou en vue de la surveillance de l'évolution de ladite tumeur, caractérisée en ce qu'elle comprend :

    (i) la préparation d'une coupe histologique à partir d'un échantillon tumoral,
    (ii) le marquage des cellules de l'échantillon obtenu en (ii) avec des anticorps spécifiques d'isoformes membranaires et solubles HLA-G, et
    (iii) l'établissement du profil d'expression HLA-G dudit échantillon, par détection des cellules marquées.

La présente invention a également pour objet une méthode d'établissement du profil d'expression HLA-G d'une tumeur solide, en vue de la sélection d'un traitement adapté à ladite tumeur et/ou en vue de la surveillance de l'évolution de ladite tumeur, caractérisée en ce qu'elle comprend :

    (i) éventuellement le marquage des cellules d'un échantillon tumoral,
    (ii) la lyse des cellules,
    (iii) la mise en contact des cellules lysées avec différents anticorps dirigés contre les antigènes HLA de classe 1, pour former, éventuellement des complexes isoforme d'HLA-G/anticorps, et
    (iv) l'établissement du profil d'expression HLA-G dudit échantillon, par détection des complexes formés à l'étape (iii).

De manière préférée, à l'étape (iii), on obtient des immunoprécipités, qui sont séparés, à l'étape (iv) par électrophorèse, transférés sur membrane et détectés.

Conformément à l'invention, lesdits anticorps sont de préférence des anticorps monoclonaux.

La recherche d'une expression des HLA-G par certaines cellules tumorales et/ou des cellules infiltrant la tumeur (macrophages, cellules dendritiques) permet de mieux évaluer le type de traitement potentiellement efficace.

En effet, la connaissance du profil de transcription de l'expression HLA-G d'une tumeur solide est cruciale pour choisir le meilleur traitement possible et suivre l'évolution de la tumeur en fonction dudit traitement.

La présente invention a également pour objet un vaccin anti-tumoral, apte à être utilisé dans les tumeurs solides exprimant au moins une isoforme d'HLA-G, caractérisé en ce qu'il est sélectionné dans le groupe constitué par des cellules tumorales autologues ou un antigène HLA-G5 soluble ou un fragment de celui-ci ; de tels vaccins induisent la formation de lymphocytes T cytotoxiques, spécifiques de tumeurs et d'anticorps anti-HLA-G.

Lorsque ledit vaccin est constitué de cellules autologues (notamment des cellules tumorales de l'individu à traiter exprimant au moins une isoforme d'HLA-G), lesdites cellules sont de préférence modifiées de manière à induire effectivement la production d'anticorps anti-HLA-G. Les cellules sont par exemple soumises à un traitement au cholestérol ou à un traitement hyperbare.

De manière avantageuse, ledit antigène HLA-G soluble ou un fragment de celui-ci est couplé à une protéine appropriée et éventuellement associé à un adjuvant tel que l'hydroxyde d'aluminium ou le phosphate de calcium.

Ledit vaccin est de préférence administré par voie sous-cutanée ou intra-dermique.

La présente invention a également pour objet une composition anti-tumorale, apte à être utilisée dans des tumeurs solides exprimant au moins une isoforme d'HLA-G, caractérisée en ce qu'elle est essentiellement constituée d'anticorps anti-HLA-G (immunothérapie passive).

La présente invention a également pour objet une composition anti-tumorale, apte à être utilisée dans des tumeurs solides exprimant au moins une isoforme d'HLA-G, caractérisée en ce qu'elle est essentiellement constituée par au moins un facteur de régulation de la transcription et/ou de l'expression des HLA-G.

Selon un mode de réalisation avantageux de ladite composition, ledit facteur de régulation est sélectionné dans le groupe constitué par les facteurs de régulation obtenus à l'aide de la méthode telle que définie ci-dessus, les facteurs antagonistes des agents d'activation des HLA-G, identifiés par les Inventeurs [interleukine-10, glucocorticoïde, interférons, action du stress (radiations, choc thermique, métaux lourds, stress oxydatif)], les acides nucléiques antisens et les inhibiteurs hormonaux de la transcription et/ou de l'expression desdites HLA-G.

La présente invention a en outre pour objet des produits contenant des anticorps anti-HLA-G et des facteurs de régulation de l'expression des HLA-G, comme produits de combinaison pour une utilisation simultanée, séparée ou étalée dans le temps, dans le traitement des tumeurs solides exprimant au moins une isoforme d'HLA-G.

Lesdits facteurs de régulation sont tels que ceux définis ci-dessus.

Outre les dispositions qui précèdent, l'invention comprend encore d'autres dispositions, qui ressortiront de la description qui va suivre, qui se réfère à des exemples de mise en oeuvre de la présente invention ainsi qu'aux dessins annexés dans lesquels :

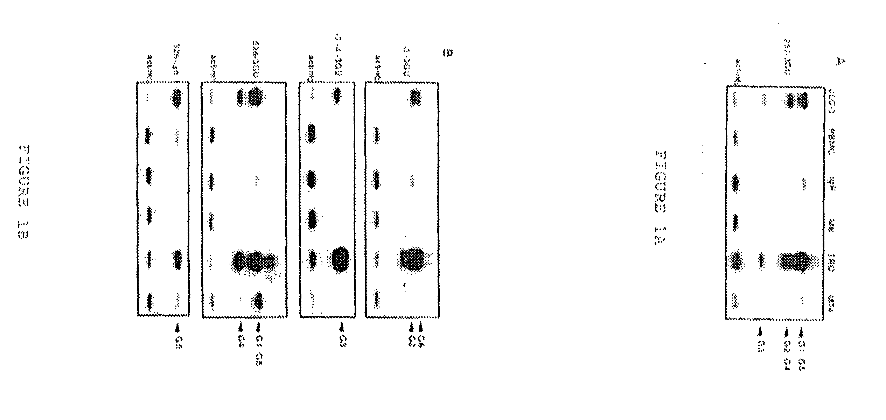

- la figure 1 illustre :

   (A) : l'analyse RT-PCR des ARNm des isoformes d'HLA-G dans les cellules de mélanome. Des amorces pan-HLA-G [amorce G.257 (exon 2) et 3G.U (extrémité 3' non-traduite)] sont utilisées pour l'amplification PCR des transcrits HLA-G correspondants aux différentes isoformes connues d'HLA-G. L'ADNc des cellules de choriocarcinome JEG-3, les trophoblastes du premier trimestre (TRO) et les cellules mononucléées du sang périphérique (PBMC) sont utilisés comme cellules contrôles respectivement pour les taux de transcription élevés et les taux de transcription basals d'HLA-G. IgR, M8, DRAN et M74 correspondent à l'amplification de l'ADNc de lignées cellulaires de mélanomes. Les bandes HLA-G spécifiques sont révélées par hybridation avec la sonde GR-spécifique, localisée au niveau de l'exon 2. Les bandes correspondant aux transcrits HLA-G1, G2, G3, G4 et G5 sont indiquées par des flèches. Les produits de la PCR, co-amplifiés au cours de la même réaction, avec les amorces de la β-actine sont détectés sur la même membrane à l'aide d'une sonde β-actine ;
   (B) : cette figure correspond à la détection RT-PCR des transcrits alternatifs dans les cellules de mélanome. L'amorce 3 est spécifique des isoformes HLA-G2 et HLA-G2 soluble (G6) qui ne possèdent pas l'exon 3. L'amorce 3.4 permet de distinguer les transcrits ARNm HLA-G3. Les amorces G.526 et 14b amplifient de manière spécifique le transcrit HLA-G5, qui correspond à la forme soluble. Les produits de la PCR, co-amplifiés dans la même réaction avec les amorces de la β-actine, sont détectés sur la même membrane par une sonde spécifique de la β-actine ;
   (C) : cette figure correspond à l'analyse RT-PCR de l'ARNm HLA-G dans des cellules de mélanome. Des amorces pan-HLA-G [amorce G.257 (exon 2) et 3G.U (extrémité 3' non-traduite)] sont utilisées pour l'amplification PCR des transcrits HLA-G correspondants aux différentes isoformes connues d'HLA-G. L'ADNc des cellules de choriocarcinome JEG-3, sont utilisés comme cellules contrôles pour les taux de transcription élevés. IgR, M8 et DRAN correspondent à l'amplification de l'ADNc de lignées cellulaires de mélanome. Les bandes HLA-G spécifiques sont révélées par hybridation avec la sonde GR-spécifique, localisée au niveau de l'exon 2. Les bandes correspondant aux transcrits HLA-G1, G2, G3, G4 et G5 sont indiquées par des flèches. Les produits de la PCR, co-amplifiés au cours de la même réaction, avec les amorces de la β-actine sont détectés sur la même membrane à l'aide d'une sonde β-actine.

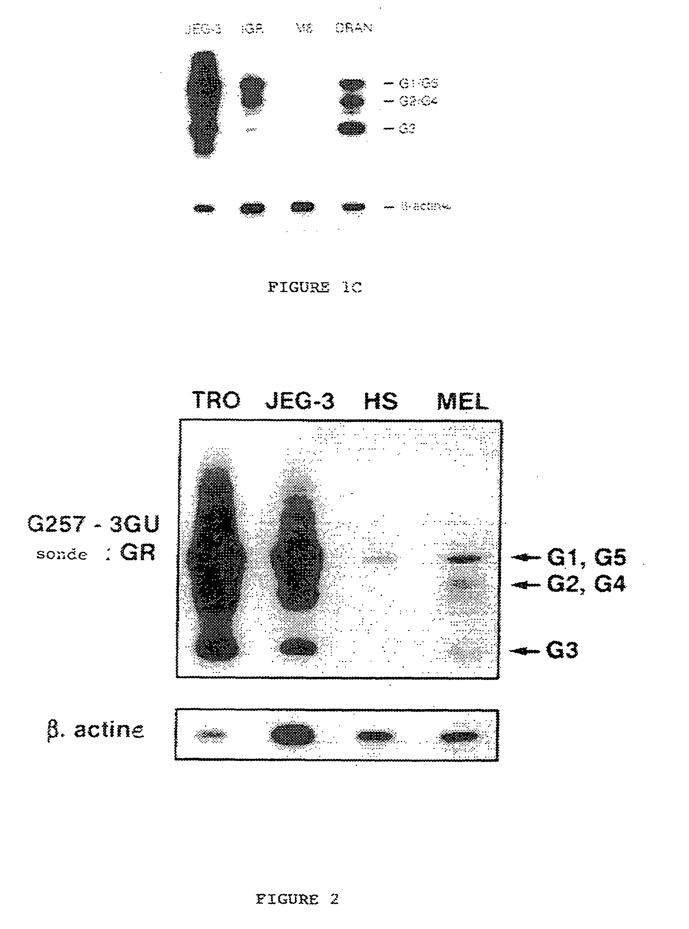

- la figure 2 illustre l'analyse RT-PCR des ARNm des isoformes d'HLA-G dans les biopsies de métastases de mélanome (analyse *in vivo* et *ex vivo* de la peau). Les amorces pan-HLA-G G.257 et 3G.U sont utilisées pour l'amplification RT-PCR des transcrits HLA-G, à partir de métastases de peau *ex vivo* (MEL) et à partir de biopsies de peau saine, du même patient (HS) ; des cellules JEG-3 et des trophoblastes du premier trimestre sont utilisés comme contrôles (taux élevé de transcription HLA-G). Les bandes spécifiques HLA-G sont révélées par hybridation avec une sonde GR-spécifique, localisée dans l'exon 2. Les bandes correspondant aux transcrits HLA-G1, G2, G3, G4 et G5 sont indiquées par des flèches.

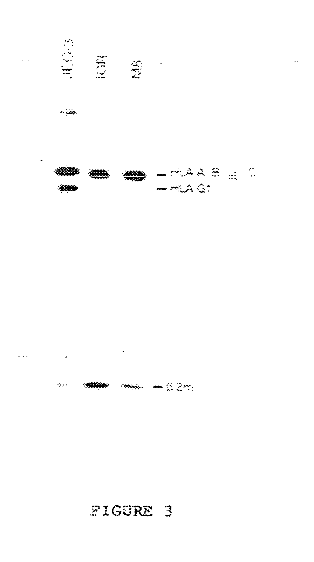

- la figure 3 illustre la détection des protéines HLA-G1 dans les cellules JEG-3 mais pas dans les cellules de mélanomes IGR et M8 à l'aide de l'anticorps monoclonal W6/32 : les protéines de surface biotinylées de mélanome et les cellules JEG-3 sont immunoprécipitées avec l'anticorps monoclonal W6/32 ; les immunoprécipités sont séparés par SDS-PAGE à 12 % et transférés sur membrane de cellulose. Les molécules de surface de classe I sont détectées avec de la peroxydase conjuguée à de la streptavidine.

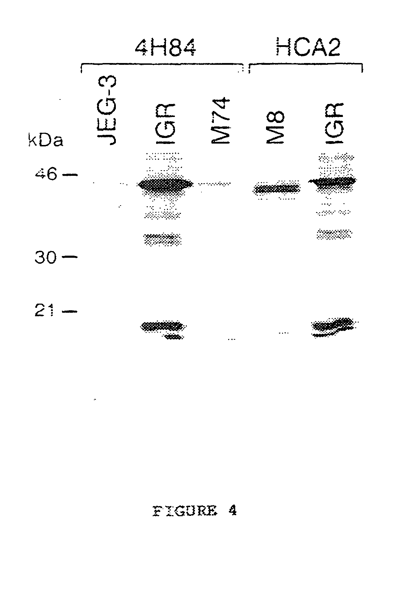

- la figure 4 illustre l'immunoprécipitation des isoformes HLA-G des cellules de mélanomes IGR par un anticorps monoclonal dirigé contre la chaîne lourde d'HLA-G libre et par les anticorps monoclonaux 4H84 et HCA2. Les cellules sont marquées pendant 30 min, immunoprécipitées avec les anticorps spécifiques et les immunoprécipités sont analysés par SDS-PAGE à 10 %. L'anticorps 4H84, qui réagit avec la chaîne lourde HLA-G (bande de 39 kDa dans les cellules JEG-3), présente des réactions croisées avec les chaînes lourdes d'HLA-A, B et/ou C (bande de 45 kDa dans toutes les cellules testées).

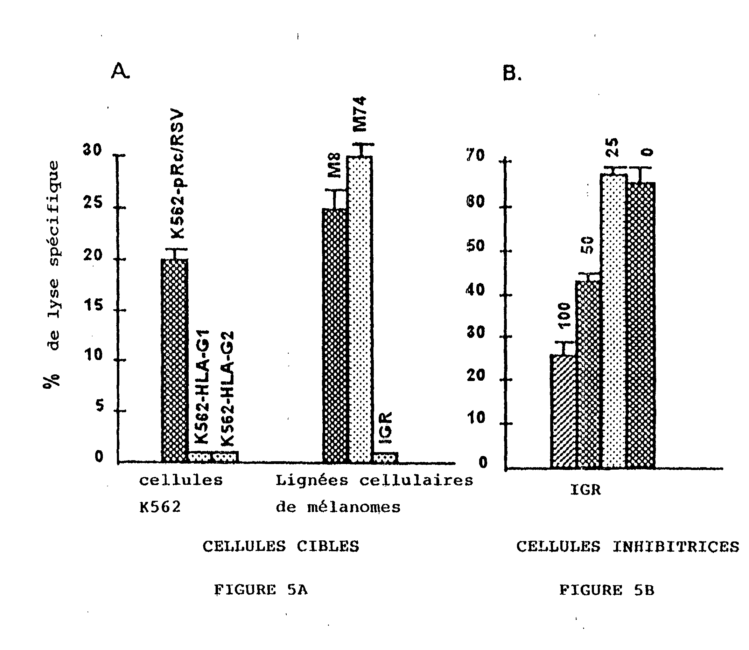

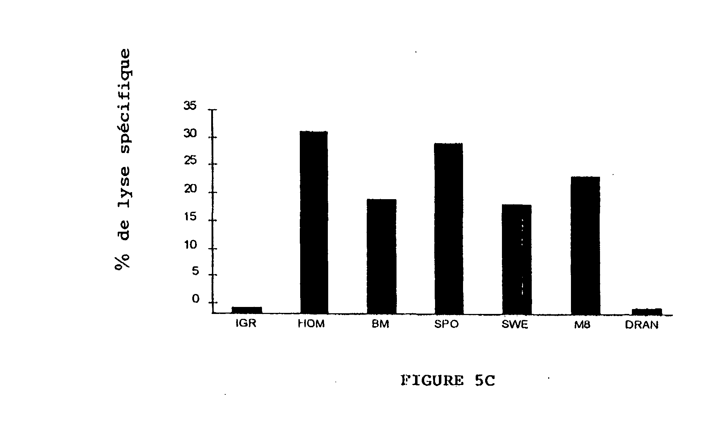

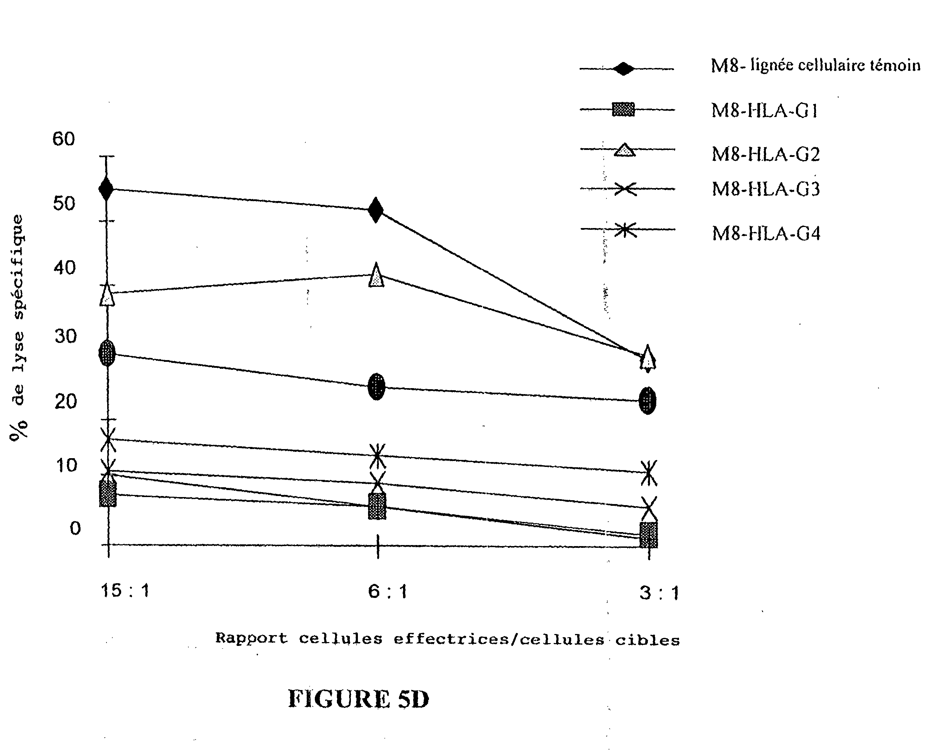

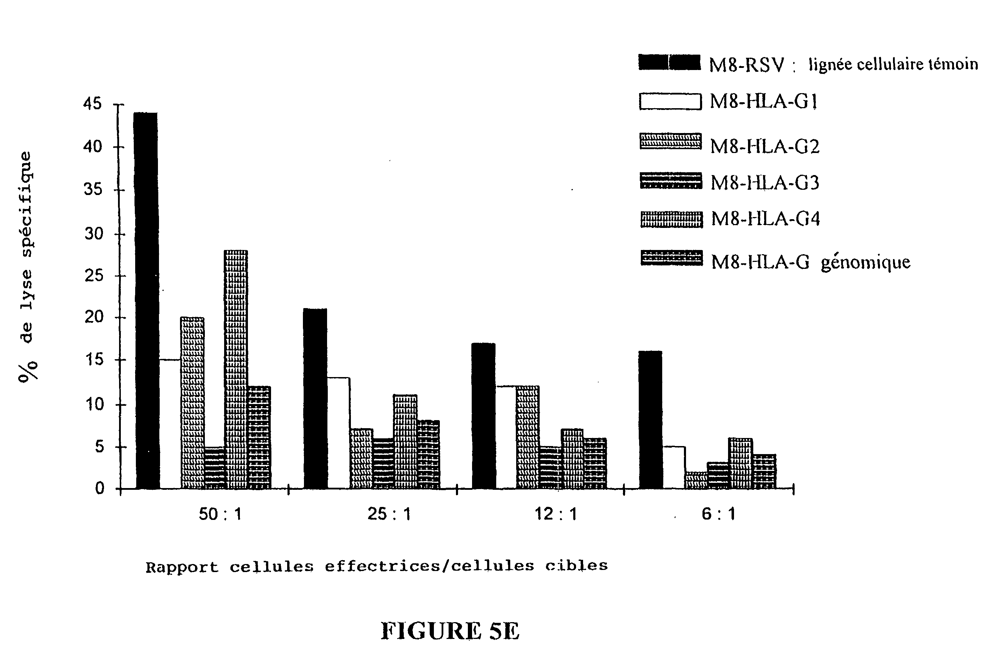

- la figure 5 illustre :

(A) : l'effet de l'expression HLA-G dans le mélanome IGR sur la sensibilité à la lyse par le clone YT2C2-PR. Les cellules K562 transfectées, soit avec le vecteur seul, soit avec le vecteur HLA-G1 contenant l'ADNc ou le vecteur HLA-G2 et les lignées M8, M74 et IGR, DRAN sont utilisées comme cellules cibles (T). Le clone YT2C2-PR est utilisé comme cellule effectrice (E) dans un rapport cellule effectrice/cellule cible (E/T) de 50/1. Les résultats sont exprimés comme le pourcentage de lyse enregistré en 4 h dans un test de libération du chrome 51. La libération spontanée n'excède jamais 10 % de la libération maximale. Cette expérience est réalisée au moins 5 fois et produit à chaque fois les mêmes résultats ;

(B) : l'inhibition de la lyse induite par le clone YT2C2-PR est due à un signal « off » transmis par les cellules IGR et DRAN. La lignée M8 est utilisée comme cellule cible (T), marquées au chrome. Le clone YT2C2-PR est utilisé comme cellule effectrice (E) dans un rapport E/T de 50:1. Les cellules IGR et DRAN sont ajoutées en tant que cellules inhibitrices dans un rapport cellule inhibitrice/cellule cible de 100, 50 et 25:1. 0 indique qu'aucune cellule IGR n'a été ajoutée dans le test ;

(C) : l'inhibition de la lyse induite par des cellules de mélanome HLA-G positives (cellules cibles T). Cette figure illustre plus particulièrement l'effet de l'expression HLA-G par des cellules de mélanome IGR et DRAN sur la sensibilité à la lyse par le clone YT2C2-PR. Plusieurs lignées cellulaires B-EBV HLA-G négatives [HOM (A3, B27, Cw1), BM (A29, B61, Cw2), SPO (A3, B7, Cw7), SWE (A2, B44, Cw5)] sont lysées par le clone YT2C2-PR. Ce clone est utilisé comme cellule effectrice (E) dans un rapport E/T de 50/1. Les résultats sont exprimés comme le pourcentage de lyse enregistré en 4 h dans un test de libération du chrome 51. La libération spontanée n'excède jamais 10 % de la libération maximale ;

(D) et (E) : ces figures montrent que les cellules tumorales HLA-G négatives M8 transfectées par les ADNc codant pour les molécules G1, G2, G3, G4 inhibent la lyse NK (figure 5E) et les réponses T cytotoxiques (figure 5D). La figure 5D comprend en abscisse les rapports cellules effectrices (E) (lignées HLA-A2 restreintes spécifiques d'un peptide de la grippe)/cellules cibles (T) (lignées M8 transfectées) et en ordonnée le pourcentage de lyse spécifique. Le Tableau ci-après correspond aux valeurs obtenues sur cette figure.

| Rapport E/T | M8-RSV | G1 | G2 | G3 | G4 | Génomique |
|---|---|---|---|---|---|---|
| 15/1 | 55 % | 8% | 39% | 12% | 17% | 30% |
| 7/1 | 52% | 6% | 42 % | 10% | 14% | 25% |
| 3/1 | 29 % | 2 % | 30% | 6% | 12% | 23 % |

La figure 5E comprend en abscisse les rapports cellules effectrices (E) (clone YT2C2-PR)/cellules cibles (T) (lignées M8 transfectées) et en ordonnée le pourcentage de lyse spécifique.

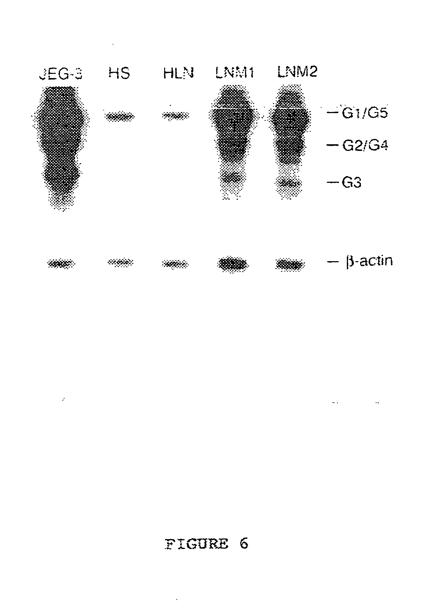

- la figure 6 illustre la détection de transcrits HLA-G dans des biopsies de mélanomes humains. Les amplifications RT-PCR sont réalisées en utilisant les amorces G.257 et G.3U précitées, à partir de biopsies de peau saine (HS) et de ganglions lymphatiques sains (HLN) d'une part et de biopsies de métastases de ganglions lymphatiques (LNM1 et LNM2). Les cellules de choriocarcinome JEG-3 sont utilisées comme cellules contrôles pour les taux de transcription élevés. Des bandes HLA-G spécifiques sont révélées par hybridation avec la sonde GR-spécifique, localisée au niveau de l'exon 2. Les bandes correspondant aux transcrits HLA-G1, G2, G3, G4 et G5 sont indiquées par des flèches. Les produits de la PCR coamplifiés au cours de la même réaction avec les amorces de la β-actine sont détectés sur la même membrane, à l'aide d'une sonde β-actine.

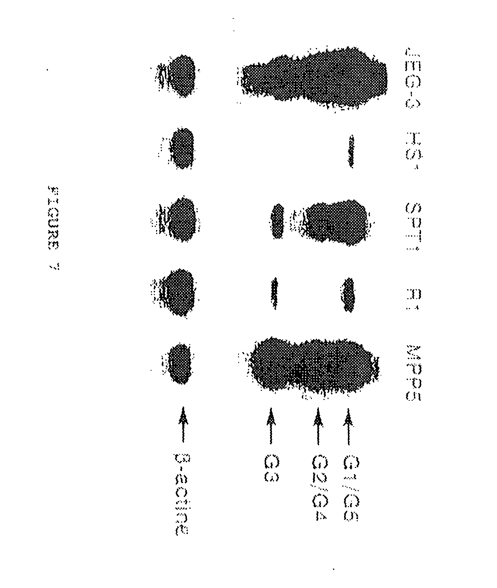

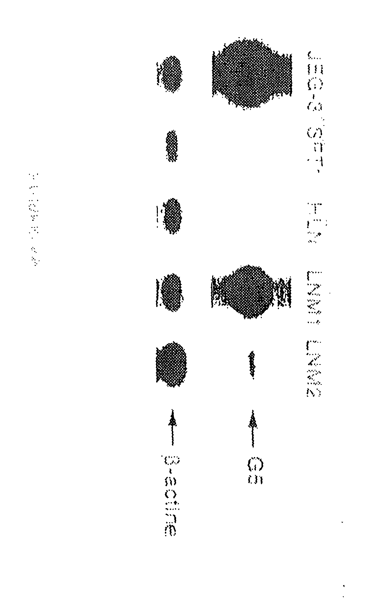

- la figure 7 illustre l'analyse RT-PCR des transcrits HLA-G dans les biopsies de tumeurs primitives de mélanomes et dans les cultures de cellules primaires MPP5 dérivées (analyse *ex vivo*). Les amorces pan-HLA-G précitées sont utilisées pour l'amplification à partir de biopsies de peau saine (HS1), de tumeurs primaires de peau (SPT1) et de tumeurs en régression (R1) obtenues à partir du même patient et à partir de cellules primaires dérivées obtenues à partir d'un tissu tumoral de peau (MPP5). Les cellules MPP5 et la biopsie SPT1 présentent des taux de transcrits HLA-G similaires. Des cellules JEG-3 sont utilisées comme contrôles de taux élevés de transcription HLA-G. Les bandes spécifiques HLA-G sont révélées par hybridation avec une sonde GR-spécifique, localisée dans l'exon 2. Les bandes correspondant aux transcrits HLA-G1, G2, G3, G4 et G5 sont indiquées par des flèches. Les produits de la PCR coamplifiés dans la même réaction avec les amorces de la β-actine sont détectés sur la même membrane par une sonde spécifique de la β-actine.

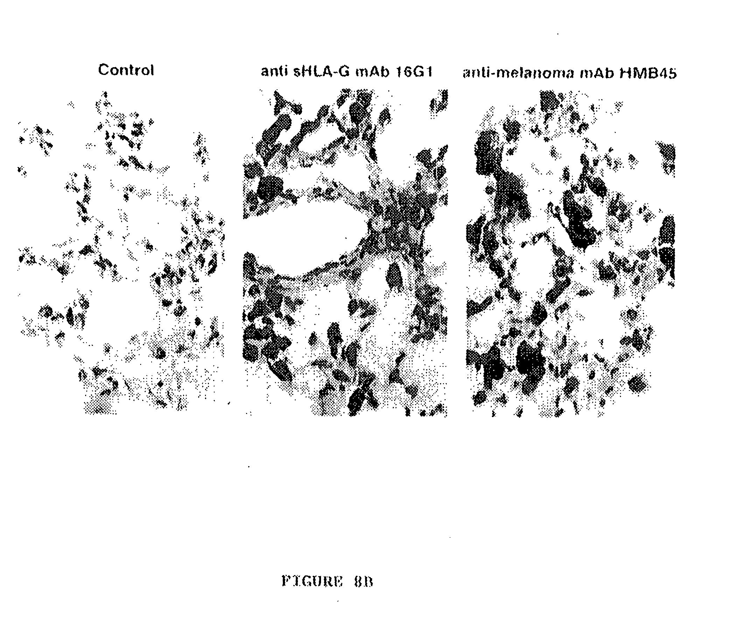

- la figure 8 illustre :

(A) la détection spécifique des transcrits HLA-G5 par RT-PCR dans des biopsies de mélanomes. L'amplification du transcrit HLA-G5 à partir de ganglions lymphatiques sains (HLN), d'une tumeur primaire de peau (SPT1) et de deux biopsies de métastases de ganglions lymphatiques (LNM1 et LNM2) est réalisée à l'aide des amorces G.526 et G.i4b. La bande correspondant au transcrit HLA-G5 est détectée par hybridation avec une sonde 14F, localisée dans l'intron 4 ; des cellules JEG-3 sont utilisées comme contrôles (taux élevés de transcription HLA-G5). La bande correspondant au transcrit HLA-G5 est indiquée par des flèches. Les produits de la PCR coamplifiés dans la même réaction avec les amorces de la β-actine sont détectés sur la même membrane par une sonde spécifique de la β-actine ;

(B) l'analyse immunohistochimique de l'expression HLA-G soluble dans la biopsie LNM1. Des sections de la biopsie LNM1 congelées et fixées sur de l'acétone sont colorées positivement avec l'anticorps anti-mélanome HMB45 (DAKO) et l'anticorps anti-HLA-G soluble 16G1, tandis que le contrôle négatif ne se colore pas en utilisant le système peroxydase anti-souris Envision (DAKO) et l'AEC comme substrat.

Il doit être bien entendu, toutefois, que ces exemples sont donnés uniquement à titre d'illustration de l'objet de l'invention, dont ils ne constituent en aucune manière une limitation.

**EXEMPLE 1 : Analyse des profils HLA-G de différentes lignées tumorales et étude de l'inhibition de la lyse par les cellules NK.**

**A/ MATERIEL ET METHODES**

**1/ Lignées cellulaires**

La lignée cellulaire érythroleucémique humaine K562 (ATCC) et la lignée cellulaire leucémique T immature (clone YT2C2-PR) à activité NK) sont maintenues dans un milieu RPMI 1640 complémenté avec du sérum de veau foetal à 10%, inactivé par la chaleur, de la L-glutamine 2mM, de la gentamicine à 1 $\mu$g/ml et de la fungizone (Sigma, Saint-Quentin, France) et cultivées à 37°C dans un incubateur, humidifié à atmosphère enrichie à 5% en $CO_2$. Les transfectants K562 sont sélectionnés dans un milieu contenant de la généticine à 1 mg/ml (G418 sulfate, Sigma).

La lignée cellulaire humaine de choriocarcinome HLA-G-positive dénommée JEG-3 (ATCC) est cultivée dans un milieu DMEM (Sigma) supplémenté avec du sérum de veau foetal à 10%, inactivé à la chaleur, des antibiotiques et de la L-glutamine 2mM. Les lignées cellulaires ne contiennent pas de mycoplasmes.

Outre les lignées précitées, on utilise :

- des lignées de mélanome IGR (HLA-A2, A3, B58/mâle), M74 (HLA-A1, A2, B8, B14/femelle), M8 (HLA-A1, A2, B12 et B40/mâle) et DRAN (HLA-A2, A3, B7, B35, CW5, CW7),
- des tissus trophoblastiques du premier trimestre, obtenus après IVG ; ces tissus sont découpés en fines lamelles et immédiatement utilisés pour extraire l'ARN, et
- des cellules mononucléées du sang périphériques (PBMC), obtenues à partir de volontaires sains et isolées sur un gradient de densité Ficoll-Hypaque 1077.

**2/ Anticorps monoclonaux**

Les anticorps suivants sont utilisés :

W6/32 : IgG2a, anti-chaînes $\alpha$ de HLA de classe I associées à la β2-m (Sigma) ; HCA2 : IgG anti-HLA-A et G et IgG anti-HLA-G, 87G, 4H84 et 16G1.

**3/ RT-PCR**

L'ARN total est extrait à partir de $10^7$ cellules à l'aide du réactif RNA NOW (Biogentex, Inc.) conformément aux recommandations du fabricant. La qualité de l'ARN est vérifiée par électrophorèse sur gel d'agarose à 1,5% dénaturant. Les ADNc sont préparés à partir de 10 $\mu$g d'ARN total traités avec de la DNAse I (Boerhinger Mannheim) en utilisant une amorce oligo-(dT)$_{12\text{-}18}$ et la transcriptase inverse M-MLV (GIBCO-BRL). Les amplifications RT-PCR HLA-G spécifiques sont réalisées en utilisant les amorces suivantes : G.257 (exon 2) et G3.U (3'UT) (Ishitani A. et al., *Proc. Natl. Acad. Sci*., 1992, **89**, 3947-3951 ; Kirszenbaum M. et al., *Proc. Natl. Acad. Sci*., 1994, **91**, 4209-4213 et Moreau P. et al., C.R. Acad. Sci, 1995, **318**, 837-842), pour détecter toutes les isoformes d'ARNm d'HLA-G. Une amplification spécifique de chaque forme d'ARNm d'HLA-G est réalisée avec les ensembles d'amorces suivants :

- G.526 (exon 3) et G3.U (3' UT) pour les isoformes G1, G4 et G5 ;
- G.526 (exon 3) et G.i4b (intron 4) pour l'isoforme G5 ;
- G.-3 (recouvrant partiellement les exons 2 et 4) et G3.U (3' UT) pour les isoformes G2 et G6 ;
- G.3-4 (recouvrant partiellement les exons 2 et 5) et G3.U (3' UT) pour l'isoforme G3.

Les ADNc des HLA classiques de classe 1 sont amplifiés comme décrit dans King et al. (*J. Immunol*., 1996, **156**, 2068-2076), en utilisant une amorce 5' unique HLA-5P2 et 3 amorces 3', HLA-3pA, HLA-3pB et HLA-3pC qui amplifient respectivement les ARNm HLA-A, HLA-B et HLA-C.

Les amorces spécifiques DRA sont décrites dans King et al. précité.

Une co-amplification de l'ADNc de β-actine est réalisée dans chaque expérience avec le test Clontech (16 cycles), de manière à évaluer les quantités comparatives d'ARN dans les échantillons. Les produits PCR sont analysés par électrophorèse sur gel d'agarose à 1% et colorés à l'aide de bromure d'éthidium. La spécificité des produits PCR est confirmée par blotting alcalin des fragments dans du NaOH 0,4 N sur des membranes de Nylon (Hybond N+, Amersham, France).

Les sondes HLA-G spécifiques sont les suivantes :

- GR spécifique de l'exon 2,
- G.647 F (5'-CCACCACCCTGTCTTTGACT: spécifique de l'exon 4),
- G.I4 F (GAGGCATCATGTCTGTTAGG : spécifique de l'intron 4), et
- G.927 F (5'-ATCATGGGTATCGTTGCTGG: spécifique de l'exon 5).

Les autres sondes sont les suivantes :

- sonde spécifique HLA-A (5'GGAGGACCAGACCCAGGACACG),
- sonde spécifique HLA-B (5'AGCTCCGATGACCACAACTGC)
- sonde spécifique HLA-C (5'TGTCCTAGCTGCCTAGGAG) et
- sonde spécifique HLA-DRA (TGTGATCATCCAGGCCGAG).

Les filtres sont exposés sur des films Kodak (Biomax) avec des écrans amplificateurs pendant 4 à 16 heures à -80°C.

### 4/ Immunoprécipitation des protéines biotinylées de surface et Western blot.

Les protéines de surface sont marquées avec de la biotine. Après lavage dans du PBS, $1,5.10^7$ cellules sont incubés dans 1 ml de PBS froid contenant 5 ml de NHS-SS-biotine (Pierce, Rockford, IL) pendant 15 min à 4°C. Les groupes actifs résiduels sont inhibés dans 50 mM de $NH_4Cl$ pendant 10 min à 4°C. Les cellules sont lysées dans 1 % de Triton X100/PBS. Les protéines précipitées avec l'anticorps W6/32 sont séparées sur SDS-PAGE à 12 %, transférées sur membrane de nitrocellulose et mises en présence d'un conjugué peroxydase de raifort-streptavidine. Après un lavage extensif de la membrane, la réaction colorée est réalisée en utilisant le réactif de détection de Western blotting ECL (Amersham, France), après quoi la membrane est exposée à un film Kodak à température ambiante.

### 5/ Essais de cytotoxicité.

L'activité cytolytique des cellules mononucléées du sang périphérique, des cellules NK et des cellules YT2C2-PR (cellules effectrices ou E) à l'encontre des transfectants HLA-G (cellules cibles ou T) est estimée à l'aide de tests de libération pendant 4 heures du chrome 51, dans lesquels les cellules effectrices sont mélangées avec $5.10^3$ de cellules cibles marquées au chrome 51 (100 µCi de $^{51}$Cr-chromate de sodium, Amersham, UK), dans différents rapports E/T, dans des plaques de microtitration dont le fond est en forme de U.

Après 4 heures à 37°C dans un incubateur humidifié contenant 5% de $CO_2$, 100 µl de surnageant sont prélevés pour un comptage par scintillation en phase liquide (Wallac 1450 Microbeta, Pharmacia, France). Le pourcentage de lyse spécifique est calculé comme suit :

$$\text{pourcentage de lyse spécifique} = [\text{cpm dans le puits expérimental - cpm de libération}$$

$$\text{spontanée)/(cpm de libération maximale - cpm de libération spontanée)}] \times 100.$$

La libération spontanée est déterminée par incubation des cellules cibles (T) marquées avec le milieu. La libération maximale est déterminée par solubilisation des cellules cibles dans de l'HCl 0,1 M. Dans toutes les expériences, la libération spontanée est inférieure à 10% par rapport à la libération maximale. Les résultats sont présentés comme des moyennes de trois échantillons. Dans les expériences dans lesquelles les anticorps monoclonaux sont utilisés pour bloquer l'interaction HLA-G-NK, les cellules cibles sont incubées avec l'anticorps monoclonal correspondant, puis lavées et incubées avec un anticorps $F(ab')_2$ de chèvre anti-souris (Jackson Immuno-research, USA) pour éviter la cytotoxicité cellulaire dépendante des anticorps (ADCC) par interaction des récepteurs pour le fragment Fc des immunoglobulines, exprimés sur les cellules NK avec le premier anticorps utilisé. Les toxicités des anticorps monoclonaux sont également vérifiées dans chaque essai et sont toujours inférieures à 3%.

## II - Résultats

### 1/ Identification des différents transcrits d'HLA-G dans des lignées cellulaires de mélanome.

Les ADNc d'HLA-G de 4 lignées cellulaires de mélanome (IGR, M8, M74 et DRAN) sont amplifiés, à l'aide des amorces précédemment décrites (A. Ishitani et al., *Proc. Natl. Acad. Sci.* USA, 1992, **89**, 3947-3951 ; M. Kirszenbaum et al., *Proc. Natl. Acad. Sci.* USA, 1994, **91**, 4209-4213), dérivées des séquences spécifiques de l'exon 2 et de la region 3' non traduite (voir Matériel et Méthodes) (figure 1).

La lignée JEG-3 de choriocarcinome et des cellules trophoblastiques, qui présentent des taux élevés de transcrits d'HLA-G, sont utilisées comme témoins positifs et les cellules mononucléées du sang périphérique (PBMC) de volontaires sains sont utilisés comme contrôle négatifs (taux faibles de transcrits HLA-G).

L'hybridation des produits de la PCR ont permis d'identifier des taux importants d'ARNm d'HLA-G dans 2 lignées cellulaires de mélanome, à savoir IGR et M74, tandis qu'aucun signal ne peut être détecté dans la lignée cellulaire de mélanome M8.

Dans les cellules JEG-3 et les trophoblastes, tous les transcrits d'HLA-G sont détectés (figures 1A et 1C).

Dans les cellules de mélanome IGR et DRAN, tous les transcrits sont également détectés par les amorces pan-HLA-G (figure 1A).

Cependant, les amorces pan-HLA-G ne permettent pas de distinguer entre les signaux HLA-G1 et HLA-G5, qui sont présents tous les deux, au niveau d'une bande correspondant à 1 000 pb, ni entre les signaux HLA-G2 et HLA-G1, qui comigrent sous la forme d'un fragment de 600 pb. Une identification RT-PCR permet d'isoler les isoformes à l'aide d'amorces spécifiques (P. Moreau et al., C. R. Acad. Sci., 1995, **318**, 837-842) (voir Matériel et Méthodes).

Les cellules IGR et DRAN expriment toutes les isoformes d'HLA-G sous forme de transcrits, HLA-G4 et HLA-G5 étant exprimés à des taux faibles (figure 1B).

Dans la lignée cellulaire de mélanome M74, les amorces pan-HLA-G détectent des bandes correspondant à HLA-G1 et HLA-G5 (1 000 pb) (signaux intenses), un signal pour HLA-G2 et G4 (600 pb), mais aucun signal pour HLA-G3 (300 pb) (figure 1A). Les amorces pour les isoformes spécifiques révèlent que dans ces cellules les isoformes G1 et G4 sont plus abondantes que dans les PBMC tandis que le taux de transcrit G5 est comparable à celui observé dans les PBMC.

Des taux faibles d'ARNm HLA-G2 et HLA-G6 (forme soluble d'HLA-G2) sont détectés dans ces cellules M74, tandis qu'une amplification spécifique du transcrit HLA-G3 confirme l'absence d'HLA-G3 observée avec les amorces pan-HLA-G dans ces cellules (figures 1A et 1B).

Aucun signal d'hybridation HLA-G n'est observé dans les cellules M8 (figures 1A et 1B).

### 2/ Analyse des protéines HLA-G dans les cellules de mélanome.

Pour déterminer si les transcrits d'HLA-G détectés dans les mélanomes sont traduits en protéines HLA-G, des études d'immunoprécipitation ont été effectuées avec différents anticorps monoclonaux anti-HLA de classe I.

La comparaison est réalisée en présence d'un contrôle positif (cellule JEG-3) et d'un contrôle négatif (cellules de mélanome M8).

Les résultats d'immunoprécipitation avec l'anticorps monoclonal W6/32 sont illustrés à la figure 3.

Avec les cellules JEG-3, l'anticorps W6/32 immunoprécipite deux protéines de 45 kDa (molécule HLA-C) et de 39 kDa (isoforme HLA-G1 liée à la membrane).

Dans les cellules IGR et M8, seulement une protéine de 45 kDa est détectée.

Des résultats similaires sont obtenus par immunoprécipitation de protéines de surface biotinylées (figure 3).

Ces données montrent que la protéine HLA-G1 n'est pas exprimée dans les cellules IGR, même si ces dernières expriment l'ARNm correspondant.

Cependant, l'absence de protéine HLA-G1 dans les cellules IGR n'exclut pas l'expression de 3 autres isoformes d'HLA-G (HLA-G2, G3 et G4).

Ces protéines ne peuvent pas être révélées par l'anticorps monoclonal W6/32, en raison de leur incapacité à s'associer avec la β2m.

Pour mettre en évidence ces protéines, l'immunoprécipitation de protéines marquées à la méthionine ($^{35}$S-méthionine) est réalisée en utilisant des anticorps monoclonaux qui reconnaissent l'HLA-G libre, l'HLA-G dénaturée et l'HLA-A (anticorps HCA2) et un épitope localisé au niveau du domaine $\alpha$1 présent dans toutes les isoformes de la protéine HLA-G (anticorps monoclonal Ig anti HLA-G).

L'anticorps monoclonal révèle la présence de la protéine HLA-G1 de 39 kDa dans les cellules JEG-3 et DRAN et son absence dans les cellules IGR (figure 4).

Des bandes additionnelles, qui migrent à 32-34 kDa et à 18 kDa, qui correspondent respectivement à la taille de la protéine HLA-G2 et/ou de la protéine HLA-G4 ou G3, sont détectées dans les cellules IGR aussi bien avec l'anticorps monoclonal Ig anti HLA-G qu'avec l'anticorps HCA2 (figure 4).

Les bandes additionnelles, spécifiques de la protéine HLA-G, ne sont pas observées dans les cellules M74 et M8 qui ne présentent pas les transcrits d'HLA-G correspondants (figure 4).

## 3/ Protection de la lignée IGR de la cytolyse induite par les cellules NK.

Les cellules YT2C2-PR sont utilisées comme cellules effectrices NK.

La lignée cellulaire IGR, qui exprime les isoformes HLA-G2 et/ou G4 et G3 et la lignée DRAN, qui exprime HLA-G1 (figure 5), abolissent la lyse induite par le clone YT2C2-PR.

La lignée cellulaire de mélanome M74, qui exprime les antigènes classiques du CMH de classe I mais qui présente un défaut sélectif dans la transcription et l'expression des isoformes HLA-G2 et HLA-G3 est lysée par le clone YT2C2-PR.

Une lyse est également observée avec la lignée cellulaire M8, qui exprime les antigènes classiques du CMH de classe I, mais qui ne transcrit aucun ARNm d'HLA-G (figures 1 et 5).

Pour montrer que seules les HLA-G sont impliquées dans cette inhibition de la lyse induite par les cellules NK, plusieurs lignées cellulaires EBV-B n'exprimant aucune HLA-G mais partageant au moins une allèle HLA-A, B ou C avec la lignée IGR sont utilisées comme cellules cibles.

Toutes ces lignées EBV-B sont lysées par le clone YT2C2-PR, montrant que les antigènes HLA-A, B et C ne sont pas impliqués dans la protection des mélanomes IGR et DRAN, de la lyse YT2C2-PR (figure 5).

Pour montrer que l'inhibition de la lyse, induite par le clone YT2C2-PR par les cellules IGR, n'est pas due à un signal transmis par cette lignée cellulaire mais est bien liée à une résistance intrinsèque de ces cellules IGR aux cellules NK, les cellules IGR ont été utilisées comme inhibiteurs, dans un test de cytotoxicité dans lequel les cellules cibles (T) sont des cellules M8 et les cellules YT2C2-PR, les cellules effectrices (E).

La figure 5 B montre que les cellules IGR inhibent de manière efficace la lyse des cellules M8 par le clone YT2C2-PR ; cette inhibition est proportionnelle au nombre de cellules IGR utilisé pour le test compétitif.

## EXEMPLE 2 : Détection des transcrits et des protéines HLA-G dans des biopsies de mélanomes.

A/ MATERIEL ET METHODES

### 1/ Échantillons tumoraux

Des biopsies sont effectuées à partir d'échantillons tissulaires de patients.

Immédiatement après le prélèvement, les échantillons sont congelés dans l'azote liquide et stockés jusqu'à l'extraction de l'ARN.

### 2/ Immunohistochimie.

Des méthodes standards sont utilisées pour réaliser l'immunohistochimie sur des coupes réalisées à partir des biopsies de mélanome, fixées à l'acétone, rincées dans du PBS et bloquées dans du sérum de lapin normal (DAKO) dans du PBS.

Les échantillons sont incubés avec l'anticorps primaire pendant 1 h à température ambiante, puis sont incubés avec un anticorps secondaire (Ig de lapin anti-souris conjugé avec du FITC) (DAKO).

Les sections sont contre-colorées avec un colorant nucléaire (DAPI, Sigma) et préparées dans un milieu convenable. La fluorescence est analysée avec un microscope confocal Io24 MRC (Bio-Rad). Les anticorps suivants sont utilisés : W6/32 : IgG2a anti-chaînes lourdes d'HLA-G de classe I associées à de la β2-micro-globuline (Sigma) et 87G : IgG2b anti-HLA-G qui détecte l'isoforme HLA-G1.

Les autres techniques sont identiques à celles de l'exemple 1.

B/ RESULTATS

**1/ Analyse de la transcription HLA-G dans des biopsies de mélanome *ex-vivo*.**

Tous les transcrits d'HLA-G sont détectés à des taux importants dans certaines biopsies de-mélanome, alors que seule la bande de 1 000 pb est détectée dans la peau humaine saine (figures 2 et 6). Ces résultats ont été confirmés sur d'autres biopsies et montrent que les taux importants de transcription observés dans les cellules de mélanome, sont spécifiques de ces derniers et non observable dans le tissu sain.

De manière plus précise, des taux élevés de transcription HLA-G sont détectés de manière spécifique dans des tumeurs primaires et dans des métastases, alors que des taux basaux de transcrits HLA-G et une absence d'expression de protéine HLA-G sont observés dans la peau saine ou dans des ganglions lymphatiques normaux (figure 6A).

L'analyse de la peau saine (HS1), des tumeurs primaires de peau (SP1) et d'un site de régression tumorale (R1) dans une tumeur primaire de peau d'un même patient permet la détection d'un taux élevé de transcrits HLA-G et d'expression de protéines au niveau du site tumoral primaire, tandis qu'aussi bien la peau saine que le site de régression tumorale présent des taux basaux de transcrits HLA-G et l'absence complète de l'expression de protéines HLA-G1 (figure 7).

Les cellules primaires en culture (MPP5) dérivées de la tumeur primaire SP1 présentent également des taux élevés de transcrits HLA-G (figure 7).

**2/ Analyse de la transcription HLA-G soluble dans des biopsies de mélanome *ex-vivo***

Une amplification spécifique des transcrits (ARNm) correspondant à l'isoforme soluble HLA-G5, dans les biopsies de mélanomes montrent que l'on détecte des taux élevés de transcrits HLA-G5 dans certaines biopsies de mélanomes dont on a montré qu'elles présentent des taux élevés de transcrits correspondant aux isoformes membranaires d'HLA-G (figure 8).

Par ailleurs, dans d'autres cas, on observe une dissociation entre les taux d'HLA-G5 et les taux des autres transcrits HLA-G : dans des biopsies de mélanomes dans lesquelles on a préalablement observé des taux élevés d'HLA-G1, G2, G3 et G4, on n'observe pas de transcrits HLA-G5.

La tumeur primaire de peau SP1 et les cellules en culture correspondantes MPP5 ainsi que les métastases de ganglions lymphatiques LNM2 présentent des taux élevés de transcrits HLA-G correspondant à des isoformes d'HLA-G liées à la membrane (figure 8), alors qu'on ne détecte pas de transcrits HLA-G5 dans le même échantillon.

3/ Analyse des protéines membranaires et solubles dans des biopsies de mélanomes

Des taux élevés de transcrits HLA-G sont corrélés à la détection spécifique de l'expression de protéine HLA-G par un anticorps monoclonal anti-HLA-G (anticorps 87G) dans des biopsies de mélanomes. En effet, l'analyse immunohistochimique de l'expression HLA-G dans une biopsie de ganglions lymphatiques métastasiques (LNM2) permet d'observer un marquage positif de LNM2 aussi bien avec l'anticorps 87G qu'avec l'anticorps W6/32, alors que le contrôle négatif constitué par la peau saine du même patient n'est pas coloré par l'anticorps anti-HLA-G.

Afin d'affiner cette étude, un anticorps qui détecte spécifiquement la protéine HLA-G soluble, l'anticorps 16G1 (Lee et al., Immunity, 1995, 3, 591-600), permet de démontrer l'expression de la protéine HLA-G soluble dans la biopsie de ganglions lymphatiques d'un patient présentant des taux élevés de transcrits HLA-G5 (figure 8).

L'analyse immunohistochimique permet le marquage de cette biopsie tandis que l'on n'observe aucune expression détectable en utilisant le même anticorps dans une biopsie de mélanomes d'un patient présentant des taux élevés des autres isoformes d'HLA-G.

En effet, l'analyse immunohistochimique de l'expression d'HLA-G soluble dans la biopsie LNM2, montre que des sections de biopsies LNM2 fixées à l'acétone sont colorées positivement avec l'anticorps anti-mélanome HMB45 (DAKO, Glostrup, ; SKELTON et al., Am. J. Dermatopathol., 1991, 13, 543-550) et l'anticorps anti-HLA-G soluble 16G1, tandis que le contrôle négatif n'est pas coloré.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1. Méthode d'établissement du profil de transcription des isoformes membranaires et solubles d'HLA-G d'une tumeur solide, en vue de la sélection d'un traitement adapté à ladite tumeur et/ou en vue de la surveillance de l'évolution de ladite tumeur, **caractérisée en ce qu'**elle comprend :

   (i) l'extraction de l'ARNm à partir d'un échantillon tumoral ;
   (ii) la transcription inverse (RT) dudit ARN ;
   (iii) les amplifications successives ou concomitantes des ADNc obtenus en (ii), en présence d'amorces spécifiques de chaque isoforme d'HLA-G et l'analyse des produits d'amplification obtenus, par électrophorèse et/ou hybridation spécifique et
   (iv) l'établissement du profil de transcription des isoformes membranaires et solubles d'HLA-G dudit échantillon.

2. Méthode d'établissement du profil d'expression des isoformes membranaires et solubles d'HLA-G d'une tumeur solide, en vue de la sélection d'un traitement adapté à ladite tumeur et/ou en vue de la surveillance de l'évolution de ladite tumeur, **caractérisée en ce qu'**elle comprend :

   (i) la préparation d'une coupe histologique à partir d'un échantillon tumoral
   (ii) le marquage des cellules de l'échantillon obtenu en (i) avec des anticorps spécifiques d'isoformes membranaires et solubles HLA-G, et
   (iii) l'établissement du profil d'expression des isoformes membranaires et solubles d'HLA-G dudit échantillon, par détection des cellules marquées.

3. Méthode d'établissement du profil d'expression des isoformes membranaires et solubles d'HLA-G d'une tumeur solide, en vue de la sélection d'un traitement adapté à ladite tumeur et/ou en vue de la surveillance de l'évolution de ladite tumeur, **caractérisée en ce qu'**elle comprend :

   (i) éventuellement le marquage des cellules d'un échantillon tumoral échantillon,
   (ii) la lyse des cellules d'un échantillon tumoral,
   (iii) la mise en contact des cellules lysées avec différents anticorps dirigés contre les antigènes HLA de classe I, pour former, éventuellement des complexes isoforme d'HLA-G/anticorps, et
   (iv) l'établissement du profil d'expression des isoformes membranaires et solubles d'HLA-G dudit échantillon, par détection des complexes formés à l'étape (iii).

4. Vaccin anti-tumoral, apte à être utilisé dans des tumeurs solides exprimant au moins une isoforme d'HLA-G, **caractérisé en ce qu'**il comprend des cellules tumorales autologues exprimant au moins une isoforme d'HLA-G.

5. Antigène HLA-G5 soluble ou un de ses fragments pour une utilisation comme vaccin anti-tumoral, apte à être utilisé dans des tumeurs solides exprimant au moins une isoforme d'HLA-G.

6. Utilisation d'un antigène HLA-G5 soluble ou d'un de ses fragments pour la préparation d'un vaccin destiné au traitement des tumeurs solides exprimant au moins une isoforme d'HLA-G.

7. Utilisation selon la revendication 6, **caractérisée en ce que** ledit antigène HLA-G5 soluble ou l'un de ses fragment est couplé à une protéine appropriée et éventuellement associé à un adjuvant tel que l'hydroxyde d'aluminium ou le phosphate de calcium.

8. Utilisation d'un anticorps anti-HLA-G pour la préparation d'un médicament anti-tumoral, destiné au traitement des tumeurs solides exprimant au moins une isoforme d'HLA-G.

9. Utilisation d'un facteur inhibiteur de la transcription et/ou de l'expression d'HLA-G pour la préparation d'un médicament anti-tumoral, destiné au traitement des tumeurs solides exprimant au moins une isoforme d'HLA-G.

**10.** Utilisation selon la revendication 9, **caractérisée en ce que** ledit facteur inhibiteur est sélectionné dans le groupe constitué par les facteurs antagonistes des agents d'activation des HLA-G, les acides nucléiques anti-sens et les inhibiteurs hormonaux de la transcription et/ou de l'expression desdites HLA-G.

**11.** Produits contenant des anticorps anti-HLA-G et des facteurs inhibiteurs de l'expression des HLA-G, comme produits de combinaison pour une utilisation simultanée, séparée ou étalée dans le temps, dans le traitement des tumeurs solides exprimant au moins une isoforme d'HLA-G.

**Patentansprüche**

**1.** Verfahren zur Feststellung des Transkriptionsprofils der Membran- und der löslichen Isoform von HLA-G eines soliden Tumors im Hinblick auf die Auswahl einer auf den Tumor abgestimmten Behandlung und/oder im Hinblick auf die Überwachung der Entwicklung dieses Tumors, **dadurch gekennzeichnet, dass** es umfasst:

(i) die Extraktion von RNA ausgehend von einer Tumorprobe;
(ii) die reverse Transkription (RT) der RNA;
(iii) aufeinander folgende oder gleichzeitige Amplifikationen der in (ii) erhaltenen cDNAs in Anwesenheit von Primern, die für jede HLA-G-Isoform spezifisch sind, und Analyse der erhaltenen Amplifikationsprodukte durch Elektrophorese und/oder spezifische Hybridisierung und
(iv) die Feststellung des Transkriptionsprofils der Membran- und der löslichen Isoform von HLA-G der Probe.

**2.** Verfahren zur Feststellung des Expressionsprofils der Membran- und der löslichen Isoform von HLA-G eines soliden Tumors im Hinblick auf die Auswahl einer auf diesen Tumor abgestimmten Behandlung und/oder im Hinblick auf die Überwachung der Entwicklung dieses Tumors, **dadurch gekennzeichnet, dass** es umfasst:

(i) die Herstellung eines histologischen Schnitts ausgehend von einer Tumorprobe;
(ii) die Markierung der Zellen der in (i) erhaltenen Probe mit für die membran- und die lösliche Isoform von HLA-G spezifischen Antikörpern und
(iii) die Feststellung des Expressionsprofils der Membran- und der löslichen Isoform von HLA-G der Probe mittels Nachweis der markierten Zellen.

**3.** Verfahren zur Feststellung des Expressionsprofils der Membran- und der löslichen Isoform von HLA-G eines soliden Tumors im Hinblick auf die Auswahl einer auf den Tumor abgestimmten Behandlung und/oder im Hinblick auf die Überwachung der Entwicklung dieses Tumors, **dadurch gekennzeichnet, dass** es umfasst:

(i) gegebenenfalls die Markierung der Zellen einer Tumorprobe;
(ii) die Lyse der Zellen einer Tumorprobe;
(iii) das In-Kontakt-Bringen der lysierten Zellen mit verschiedenen Antikörpern, die gegen die HLA-Antigene der Klasse I gerichtet sind, um gegebenenfalls HLA-G-Isoform/Antikörper-Komplexe zu erhalten, und
(iv) die Feststellung des Expressionsprofils der Membran- und der löslichen Isoform von HLA-G der Probe mittels Nachweis der im Schritt (iii) gebildeten Komplexe.

**4.** Anti-Tumor-Impfstoff, der an die Verwendung in soliden Tumoren angepasst ist, die mindestens eine Isoform von HLA-G exprimieren, **dadurch gekennzeichnet, dass** er autologe Tumorzellen umfasst, die mindestens eine Isoform von HLA-G exprimieren.

**5.** Lösliches HLA-G5-Antigen oder eines seiner Fragmente zur Verwendung als Anti-Tumor-Impfstoff, der an die Verwendung in soliden Tumoren angepasst ist, die mindestens eine Isoform von HLA-G exprimieren.

**6.** Verwendung eines löslichen HLA-G5-Antigens oder eines seiner Fragmente zur Herstellung eines Impfstoffs, der zur Behandlung solider Tumoren, die mindestens eine Isoform von HLA-G exprimieren, bestimmt ist.

**7.** Verwendung nach Anspruch 6, **dadurch gekennzeichnet, dass** das lösliche HLA-G5-Antigen oder eines seiner Fragmente mit einem geeigneten Protein gekoppelt und gegebenenfalls mit einem Adjuvans, wie Aluminiumhydroxid oder Calciumphosphat, zusammengebracht wird.

**8.** Verwendung eines Anti-HLA-G-Antikörpers zur Herstellung eines Anti-Tumor-Medikaments, das zur Behandlung solider Tumoren, die mindestens eine Isoform von HLA-G exprimieren, bestimmt ist.

9.  Verwendung eines Inhibitors der Transkription und/oder der Expression von HLA-G zur Herstellung eines Anti-Tumor-Medikaments, das zur Behandlung solider Tumoren, die mindestens eine Isoform von HLA-G exprimieren, bestimmt ist.

10. Verwendung nach Anspruch 9, **dadurch gekennzeichnet, dass** der Inhibitor aus der Gruppe ausgewählt ist, die aus den Antagonisten und Aktivierungsmitteln von HLA-G, Antisense-Nucleinsäuren und hormonellen Inhibitoren der Transkription und/oder Expression dieser HLA-G besteht.

11. Produkte, die Anti-HLA-G-Antikörper und Inhibitoren der Expression von HLA-G enthalten, als Kombinationsprodukte zur gleichzeitigen, getrennten oder zeitlich ausgedehnten Verwendung zur Behandlung solider Tumoren, die mindestens eine Isoform von HLA-G exprimieren.

**Claims**

1.  Method of establishing the transcription profile of the membrane-bound and soluble isoforms of HLA-G of a solid tumour in order to select an appropriate treatment for the said tumour and/or in order to monitor the progress of the said tumour, **characterised in that** it comprises:

    (i) the extraction of mRNA from a tumour sample;
    (ii) the reverse transcription (RT) of the said RNA;
    (iii) the successive or concomitant amplification of the cDNA obtained in (ii), in the presence of primers specific to each HLA-G isoform, and the analysis of the resulting amplification products, by electrophoresis and/or specific hybridisation and
    (iv) the establishment of the transcription profile of the membrane-bound and soluble isoforms of HLA-G of the said sample.

2.  Method of establishing the expression profile of the membrane-bound and soluble isoforms of HLA-G of a solid tumour in order to select an appropriate treatment for the said tumour and/or in order to monitor the progress of the said tumour, **characterised in that** it comprises:

    (i) the preparation of a histological section from a tumour sample;
    (ii) the labelling of the cells of the sample obtained in (i) with antibodies specific to membrane-bound and soluble isoforms of HLA-G, and
    (iii) the establishment of the expression profile of the membrane-bound and soluble isoforms of HLA-G of the said sample, by detecting the labelled cells.

3.  Method of establishing the expression profile of the membrane-bound and soluble isoforms of HLA-G of a solid tumour in order to select an appropriate treatment for the said tumour and/or in order to monitor the progress of the said tumour, **characterised in that** it comprises:

    (i) optionally labelling of the cells of a tumour sample;
    (ii) the lysis of the cells of a tumour sample;
    (iii) the reaction of the lysed cells with different antibodies directed against class 1 HLA antigens in order to form, possibly, HLA-G isoform/antibody complexes, and
    (iv) the establishment of the expression profile of the membrane-bound and soluble isoforms of HLA-G of the said sample, by detecting the complexes formed in stage (iii).

4.  Anti-tumour vaccine which can be used in solid tumours expressing at least one HLA-G isoform, **characterised in that** it comprises autologous tumour cells expressing at least one HLA-G isoform.

5.  Soluble HLA-G5 antigen or one of its fragments for use as an anti-tumour vaccine which can be used in solid tumours expressing at least one HLA-G isoform.

6.  Use of a soluble HLA-G5 antigen or one of its fragments for the preparation of a vaccine intended for the treatment of solid tumours expressing at least one HLA-G isoform.

7. Use according to claim 6, **characterised in that** the said soluble HLA-G5 antigen or one of its fragments is coupled with an appropriate protein and optionally is associated with an adjuvant such as aluminium hydroxide or calcium phosphate.

8. Use of an anti-HLA-G antibody for the preparation of an anti-tumour drug intended for the treatment of solid tumours expressing at least one HLA-G isoform.

9. Use of a factor inhibiting the transcription and/or the expression of HLA-G for the preparation of an anti-tumour drug intended for the treatment of solid tumours expressing at least one HLA-G isoform.

10. Use according to claim 9, **characterised in that** the said inhibitor is selected from the group formed by antagonists for HLA-G activating agents, antisense nucleic acids and hormonal inhibitors for the transcription and/or the expression of the said HLA-G.

11. Products containing anti-HLA-G antibodies and factors inhibiting the expression of HLA-G as combined products for simultaneous, separate or staged use in the treatment of solid tumours expressing at least one HLA-G isoform.

FIGURE 1A

FIGURE 1B

FIGURE 1C

G257 - 3GU
sonde : GR

G1, G5
G2, G4
G3

β. actine

FIGURE 2

FIGURE 3

FIGURE 4

A.

% de lyse spécifique

K562-pRc/RSV

M8

M74

K562-HLA-G1

K562-HLA-G2

IGR

cellules
K562

Lignées cellulaires
de mélanomes

CELLULES CIBLES

FIGURE 5A

B.

IGR

100

50

25

0

CELLULES INHIBITRICES

FIGURE 5B

EP 1 054 688 B1

FIGURE 5C

FIGURE 5D

**FIGURE 5E**

EP 1 054 688 B1

FIGURE 6

FIGURE 7

Control    anti sHLA-G mAb 16G1    anti-melanoma mAb HMB45

FIGURE 8B